# EUROPÄISCHE PATENTANMELDUNG

(11) **EP 1 170 593 A2**
(43) Veröffentlichungstag der Anmeldung: **09.01.2002**
(21) Anmeldenummer: 01250196.1
(22) Anmeldetag: 31.05.2001
(51) Int. Cl.: G01N 33/68, G01N 33/72

(54) **Feste Reagenzmischung, Testkit und Verfahren zur Bestimmung von Gesamteiweiss und Globulinen sowie von Hämoglobin in biologischen Flüssigkeitsproben**

(30) Priorität: 19.06.2000 DE 10030193; 19.06.2000 DE 20010763 U
(71) Anmelder: Levine, Felix, 41236 Mönchengladbach (DE)
(72) Erfinder: Levine, Felix, 41236 Mönchengladbach (DE)
(74) Vertreter: Ziebig, Marlene, Dr. Dipl.-Chem.

(57) **Zusammenfassung**

Die Erfindung betrifft einen Testkit zur Bestimmung von Gesamteiweiß und von Globulinen sowie von Hämoglobin unmittelbar in einer biologischen Flüssigkeitsprobe, insbesondere im Urin, wobei er die zur Bestimmung notwendige, erfindungsgemäße Reagenzmischung in einer festen Formulierung umfasst. Die feste Reagenzmischung enthält neben an sich üblichen Reagentien zur Gesamteiweißfällung und zur visuellen Beurteilung des Resultates der Fällungsreaktion als weiteres Fällungsreagenz Kupfersulfat. Zusätzlich enthält sie mindestens ein Polysaccharid und gegebenenfalls mindestens ein Monosaccharid. Die feste Formulierung kann z.B. in Form eines Granulates oder einer Tablette vorliegen. Neben dem Testkit und der Reagenzienmischung in fester Form betrifft die Erfindung ein Verfahren zum Nachweis von Gesamteiweiß und von Globulinen sowie von Hämoglobin in einer biologischen Flüssigkeitsprobe mit der erfindungsgemäßen Reagenzmischung.

## Beschreibung

Die Erfindung betrifft einen Testkit zur Bestimmung von Gesamteiweiß und von Globulinen sowie von Hämoglobin unmittelbar in einer biologischen Flüssigkeitsprobe, insbesondere im Urin, wobei er die zur Bestimmung notwendige, erfindungsgemäße Reagenzmischung in einer festen Formulierung umfasst. Die feste Reagenzmischung enthält neben an sich üblichen Reagentien zur Gesamteiweißfällung und zur visuellen Beurteilung des Resultates der Fällungsreaktion als weiteres Fällungsreagenz Kupfersulfat. Zusätzlich enthält sie mindestens ein Polysaccharid und gegebenenfalls mindestens ein Monosaccharid. Die feste Formulierung kann z.B. in Form eines Granulates oder einer Tablette vorliegen. Neben dem Testkit und der Reagenzienmischung in fester Form betrifft die Erfindung ein Verfahren zum Nachweis von Gesamteiweiß und von Globulinen sowie von Hämoglobin in einer biologischen Flüssigkeitsprobe mit der erfindungsgemäßen Reagenzmischung.

Methoden zur qualitativen und quantitativen Bestimmung von Proteinen (Gesamteiweiß) im Urin, in Plasma, Serum, Liquor und in anderen biologischen Flüssigkeiten sind zahlreich bekannt und diagnostisch relevant. Die Proteine können durch Fällungs- und Farbreaktionen nachgewiesen werden. Solche Methoden dienen dem Nachweis zahlreicher Krankheiten. Der Nachweis der Proteinurie (Eiweißausscheidung im Urin) dient vor allem zur Diagnose verschiedener Nierenerkrankungen.

Der Eiweißnachweis kann qualitativ und halbquantitativ mit Teststreifen erfolgen, die in der Regel ein Reagenz enthalten, das mit dem Eiweiß eine Farbreaktion bildet. Diese Teststreifen haben den Nachteil, dass sie durch Licht, Feuchtigkeit und hohe Temperaturen empfindlich und unbeständig, also nicht lagerfähig sind, wodurch eine Auswertung nach längeren Zeiträumen nicht mehr möglich ist. Außerdem besitzen sie eine ungenügende analytische Empfindlichkeit.

Andere qualitative, halbquantitative oder quantitative Nachweise erfolgen z.B. durch Zugabe von Sulfosalicylsäure zum Urin bzw. mit der Biuretreaktion nach Fällung mit Trichloressigsäure oder Perchlorsäure, gegebenenfalls unter Farbstoffzusätzen. Bekannte Fällungsreagentien sind neben organischen Säuren u.a. auch Schwermetallsalze, Aceton, Alkohol, Ammoniumsalze und dergleichen.
Ein Testsystem zur Eiweißbestimmung im Urin ist in dem russischen Patent RU 2 077 060 C1 beschrieben. Dieses Testsystem, das zur Fällung des Eiweisses mit einem Reagenz, das gegebenenfalls mit einem in Wasser unlöslichen Bestandteil verbunden ist, eingesetzt wird, enthält das Reagenz zur Eiweißfällung als ein dichtes, festes Granulat mit einem spezifischen Gewicht größer 1.

Die bekannten Testsysteme haben jedoch sämtlich den Nachteil, dass man nur den Gesamteiweißgehalt bestimmen kann. Die Bestimmung des Gesamteiweißgehaltes erlaubt wiederum weder eine deutliche Diagnose noch die Feststellung der jeweiligen Schwere der Erkrankung. Dies ist aber zur differenziellen Diagnostik so z.B. von verschiedenen Arten der Nierenerkrankungen, Leukämien, Myelom aber auch bei weiteren Erkrankungen wichtig. So spielen neben dem Gesamteiweißgehalt besonders die Globuline u.a. bei der Feststellung der Nierenselelektivität eine bedeutende Rolle. Auch der Nachweis von Hämoglobin im Urin ist bei Vorliegen einer Hämaturie infolge von Schädigungen der Harnwege z.B. durch Nieren- oder Blasentumore sowie anderen Nierenerkrankungen ein wichtiger Indikator. Für weitere diagnostische Hinweise müssen die einzelnen Proteine im Gesamteiweiß jedoch erst (z.B. mittels Elektrophorese nach ihrem Molekulargewicht) weiter aufgetrennt werden. Ein weiterer Nachteil von bekannten Testsystemen ist eine für Laboranten unbequeme und gefährliche Handarbeit, weil jeder Hautkontakt mit den Reagentien vermieden werden sollte. Desweiteren können Trübungen entstehen, die das Analyseergebnis verschlechtern.

Aufgabe der Erfindung war es deshalb, ein einfach anwendbares Testsystem bereitzustellen, das es ohne größeren technischen Aufwand gestattet, neben dem Gesamteiweiß die Globuline und gegebenenfalls Hämoglobin als weitere diagnostische Parameter auszuwerten.

Erfindungsgemäß wird die Aufgabe durch einen Testkit gelöst, der eine Reagenzmischung in einer festen, dichten Formulierung umfasst, mit welcher Gesamteiweiß und Globuline sowie gegebenenfalls Hämoglobin ausgefällt werden. Die in fester Form vorliegende Reagenzmischung kann sich fixiert oder lose in einem Testreagenzgefäß, wie z.B. einem Reagenzglas, einer Titerplatte oder ähnlichem, befinden. Die feste Reagenzmischung kann beispielsweise als Tablette oder Granulat formuliert sein. Bei Verwendung eines dichten, festen Granulates weist dieses Granulat ein spezifisches Gewicht größer 1 auf. Der Testkit stellt eine visuelle Beurteilung des Resultates der Fällungsreaktion sicher.

Die erfindungsgemäße feste Reagenzmischung enthält neben an sich üblichen Fällungsreagentien, wie beispielsweise Sulfosalicylsäure und Natriumchlorid, als weiteres Fällungsreagenz Kupfersulfat und darüber hinaus mindestens ein Polysaccharid, wodurch insbesondere der Gesamteiweißgehalt und der Globulingehalt bestimmt werden. In einer bevorzugten Ausführungsvariante der Erfindung enthält die Reagenzmischung weiterhin mindestens ein Monosaccharid, wodurch man einen Unterschied zwischen verschiedenen Proteinpräzipitaten, z.B. Immunoglobulin- und Hämoglobinpräzipitaten erreichen und feststellen kann.

Gemäß der Erfindung enthält die feste Reagenzmischung die Reagentien vorzugsweise mit folgenden Gew.%:

| | |
|---|---|
| Fällungsreagentien (mit Kupfersulfat) | 85-95 Gew.%, |
| Polysaccharid | 5-15 Gew.%. |

Enthält die Reagenzmischung zusätzlich ein Monosaccharid, so sind die folgenden prozentualen Anteile bevorzugt:

| | |
|---|---|
| Fällungsreagentien (mit Kupfersulfat) | 70-90 Gew.%, |
| Polysaccharid | 5-15 Gew.%, |
| Monosaccharid | 5-15 Gew.%. |

Als erfindungsgemäß bevorzugtes Fällungsreagenz dient eine Mischung aus einer festen organischen Säure und einem anorganischen Salz in Kombination mit Kupfersulfat. Die organische Säure, z.B. Sulfosalicylsäure, macht dabei vorzugsweise 80-95 Gew.% aus, das anorganische Salz (z.B. NaCl) vorzugsweise 4-15 Gew.%, und das Kupfersulfat ist zu 1-5 Gew.% enthalten.

Als Polysaccharid ist bevorzugt ein lösliches Dextran enthalten. Als Monosaccharid wird bevorzugt eine Glucose, insbesondere D-Glucose, verwendet.

Die Reagenzmischung wird als feste Formulierung, z.B. als Granulat oder Tabelle, bereitgestellt. Zur Durchführung der Fällungsreaktionen befindet sich die feste Reagenzmischung (immobilisiert oder lose) in einem durchsichtigen Testreagenzgefäß, wie z.B, einem Reagenzglas, einem Plastikflakon oder in einer Titerplatte. Die festen Formulierungen, so z.B. die Granulate, weisen keine harmonische geometrische Form auf. So kann z.B. das Granulat u.a. in Form von Kugeln, Stäbchen, Zylindern, Linsen o.ä. vorliegen. In einer bevozugten Variante stellt die Reagenzmischung ein Granulat in Linsenform dar, wobei für die Linsen ein Durchmesser von 2-8 mm und eine Dicke von 1-4 mm bevorzugt ist. Eine Linsenform begünstigt z.B. eine gute Lösbarkeit des Granulates in der zu untersuchenden biologischen Flüssigkeit.

Die Reagenzmischung wird nach an sich bekannten Verfahren für das Granulieren oder Tablettieren chemischer Mischungen hergestellt. Es ist auch möglich, z.B. eine gesättigte Lösung des Fällungsreagenzes, die auch das Polysaccharid und das Kupfersulfat sowie gegebenenfalls das Monosaccharid enthält, auf eine glatte Plastikoberfläche oder in ein durchsichtiges Testreagenzgefäß zu tropfen und bei 10 bis 20 °C auszutrocknen. Zur Herstellung der Linsenform des Granulates wird z.B. eine o.g. Lösung auf eine Plastikplatte mit Vertiefungen, die eine gewünschte Linsenform besitzen, getropft und ausgetrocknet.

Der Testkit kann die so hergestellte feste Formulierung verschlossen bereits in dem Testreagenzgefäß enthalten, in dem es lose oder immobilisiert vorliegen kann. Es ist auch möglich, daß die feste Formulierung lose in einem anderen geschlossenen Glas- oder Plastikflakon bereitgestellt wird und der Testkit dieses Gefäß mit der Formulierung und das leere Testreagenzgefäß umfasst.

Gegenstand der Erfindung ist auch ein Verfahren zum Nachweis von Gesamteiweiß und Globulinen sowie gegebenenfalls von Hämoglobin in biologischen Flüssigkeitsproben, insbesondere im Urin.

In einer Ausführungsvariante ist das Verfahren zur Eiweißbestimmung dadurch gekennzeichnet, dass man die erfindungsgemäße Reagenzmischung unter Verwendung von Kupfersulfat und Polysaccharid in die zu prüfende biologische Flüssigkeit gibt und nach einer kurzen Einwirkungszeit von maximal 10 Minuten den gesamten Eiweißgehalt anhand des entstehenden Niederschlages, das heißt anhand des entstehenden weißen Eiweißbelages über der festen Reagenzmischung (z.B. dem Granulat oder der Tablette) bestimmt. Vorzugsweise werden dazu 0,5-1 ml der zu bestimmenden Probe verwendet.

Anschließend wird die feste Reagenzmischung in der zu untersuchenden biologischen Flüssigkeit homogen verteilt.

Gegebenenfalls wird dazu die Mischung aus Granulat und biologischer Flüssigkeit mechanisch behandelt, z.B. gerührt oder geschüttelt, bis die feste Formulierung gelöst in der zu untersuchenden Flüssigkeit vorliegt.

Man läßt diese homogene Lösung dann mindestens 30 Minuten bis 1,5 Stunden ruhig stehen. Bei Anwesenheit von Globulinen kommt es nach dieser Zeit in der Lösung bereits zu einer Flockenbildung. Innerhalb kurzer Zeit nach Auflösung der festen Formulierung bilden die gegebenenfalls vorhandenen Globuline dann einen Niederschlag (Sedimentablagerung), der halbquantitativ mit einer Foto- oder Computerscan-Skala bewertet werden kann (Abb. A).

Die erfindungsgemäße feste Reagenzmischung ermöglicht in ihrer Zusammensetzung die Bestimmung sowohl des Gesamteiweißes als auch der Globuline unmittelbar nacheinander in einer Probe der zu untersuchenden biologischen Flüssigkeit. Die erfindungsgemäße Reagenzmischung ermöglicht es, die Verschiedenheiten zwischen den Sedimentarten des Gesamteiweiß- und Globulin-Niederschlages zu erkennen und in einem zweiten Schritt auch den Globulingehalt festzustellen.

In einer weiteren Ausführungsform des erfindungsgemäßen Verfahrens können Gesamteiweiß, Globuline und Hämoglobin bestimmt werden, indem man z.B. 0,5 ml der zu prüfenden biologischen Flüssigkeit, vorzugsweise Urin, in ein Testreagenzglas mit der erfindungsgemäßen festen Reagenzmischung, die neben dem Kupfersulfat sowohl ein Polysaccharid als auch ein Monosaccharid enthält, gießt, es 5-10 Minuten in Ruhe stehen läßt, und den gesamten Eiweißgehalt anhand des entstehenden Niederschlages (weißer Eiweißring über dem Reagenz) bestimmt.

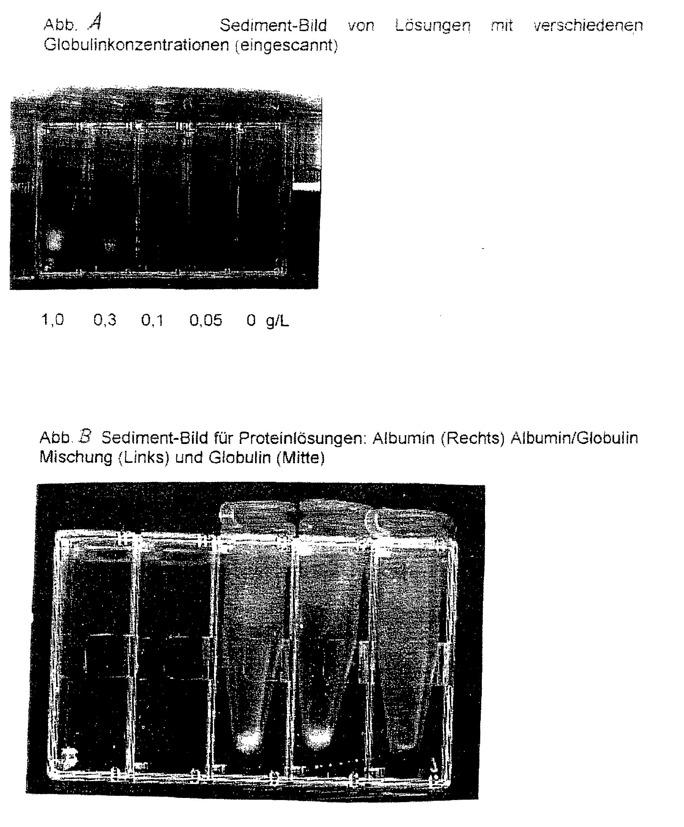

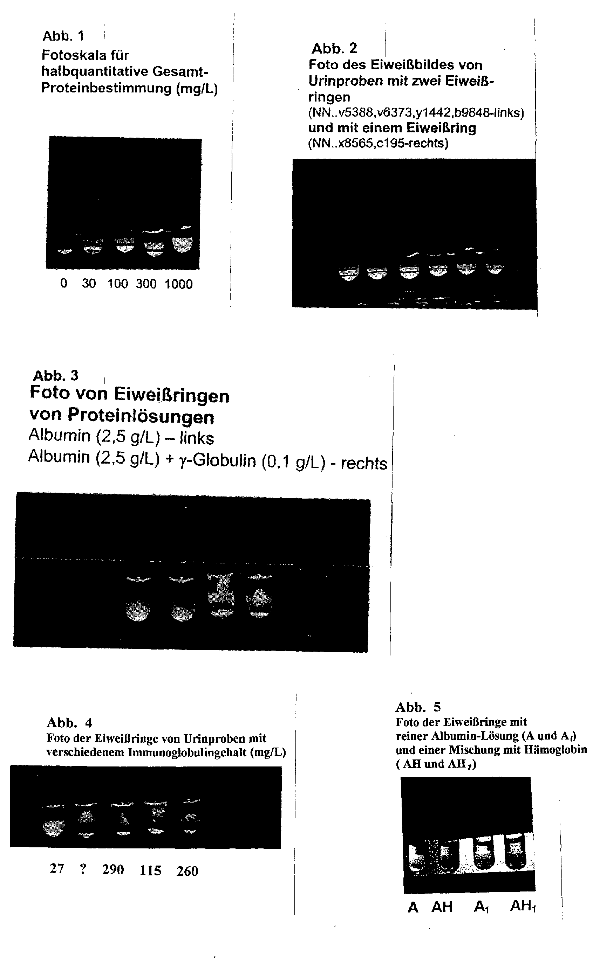

Den Gesamteiweißgehalt kann man halbquantitativ mit einer Fotoskala bestimmen, vgl. Abb. 1. Wenn noch ein zweiter Eiweißring (über dem ersten ) entsteht, vgl. Abb. 2, ist dies gleichzeitig ein Indiz dafür, dass ein erhöhter Alpha-1-Mikroglobulingehalt vorliegt.

Danach läßt man die Probe mit dem Gesamteiweißgehalt mindestens weitere 30 Minuten bis 1,5 Stunden ruhig stehen. Bei Anwesenheit von Immunoglobulinen verwandelt sich nach dieser Zeit der Eiweißring in die sogenannte "Gebirgsform", d.h. das Immunoglobulinpräzipitat taucht aus dem gesamten Eiweißniederschlag teilweise auf. Gegebenenfalls wird zu einer Beschleunigung der Immunoglobulinpräzipitätsreaktion auf 20 - 50 Minuten der Probe 0,1 ml Aceton zugegeben (Abb.3). Dabei kann das Aceton sowohl am Beginn der Fällung als auch nach der Gesamteiweißfällung zugegeben werden, wobei eine Beschädigung der Eiweißringe zu vermeiden ist.

Befindet sich außerdem Hämoglobin (beispielsweise 300 µl/1 l Blut) in der zu prüfenden Probe, wird gleichzeitig ein roter Ring über der erfindungsgemäßen Reagenzmischung sichtbar. Dies ist ebenfalls in den 30 Minuten bis 1,5 Stunden der Ruhephase deutlich zu beobachten (Abb. 5).

Damit können mit der erfindungsgemäßen festen Reagenzmischung sowohl Gesamteiweiß als auch Globuline und Hämoglobin unmittelbar nacheinander in nur einer Probe der zu untersuchenden biologischen Flüssigkeit bestimmt werden.

Die erfindungsgemäße Reagenzmischung ermöglicht es, die Verschiedenheiten zwischen den Sedimentarten des Gesamteiweiß - und von anderen Protein-Niederschlägen (Alpha-1-Mikroglobulin, Immunoglobulin und auch Hämoglobin) zu erkennen.

Der Testkit und die feste Reagenzmischung sind inbesondere zur in vitro Diagnostik in medizinischen (z. B. Notarztdienst) und tiermedizinischen Einrichtungen sowie Laboratorien einsetzbar und zur Krankendiagnostik geeignet.

Die Handhabbarkeit ist äußerst einfach, da die Reagentien in einem kompletten System vorliegen. Dadurch wird der Test nur mit wenigen Sekunden manueller Arbeitszeit durchführbar (ca. 5 Sekunden).

Der erfindungsgemäße Testkit bzw. die erfindungsgemäße feste Reagenzmischung sind für erste differenzielle diagnostische Zwecke, insbesondere bei Nierenerkrankungen, einsetzbar, insbesondere beim ersten Schritt - dem Feststellen einer Nierenselektivität. Desweiteren kann damit auch der Krankheitsverlauf kontrolliert werden.

Das Nachweisverfahren ist halbquantitativ durchführbar, mit Fotoskala oder Scanner-Skala können die Ergebnisse objektiv bewertet werden. Das Analysenergebnis (ein Proteinsedimentsbild ) kann entweder mittels einer Fotografie (z.B. mit normalen und digitalen Fotogeräten erstellt) oder auch direkt vom Testreagenzglas per Computernetz weiteren Fachleuten zur kompetenten Beurteilung übermittelt werden.

Anschließend wird die Erfindung an Ausführungsbeispielen näher erläutert.

### Beispiel 1

Der Testkit hatte die folgende Zusammensetzung:

| | |
|---|---|
| Sulfosalicylsäure | 80 Gew.% |
| NaCl | 7 Gew.% |
| Kupfersulfat | 4 Gew.% |
| Dextran | 9 Gew.% |

### a) Vergleichende Eiweißbestimmung mit reinen Proteinlösungen

Albumin aus menschlichem Serum und γ-Globulin wurden in normalem Urin mit einer Konzentration von 0,3 g/l gelöst. Es wurden drei Proben verglichen:
Probe 1: Albumin-Lösung
Probe 2: Globulin-Lösung
Probe 3: Albumin/Globulin-Mischung (1:1)

Zu 0,5 ml jeder Lösung wurde der Testkit in Form eines Granulates gegeben. Anschließend wurde das Analysenverfahren wie beschrieben durchgeführt.

Ergebnis:
1. In allen drei Proben waren nach 5 bis 10 Minuten Eiweißringe über dem Granulat zu sehen, d.h. in jeder Probe ist Eiweiß vorhanden.
2. Die Granulate wurden durch Schütteln in allen Proben gelöst, in den Proben 2 und 3 konnte man nach 30 bzw. nach 45 Minuten eine deutliche Ausflockung erkennen, nach 3 Stunden war jweils ein typisches Sediment unten im Reagenzglas zu verzeichnen. Probe 1, in der kein Globulin enthalten war, zeigt lediglich eine homogene Trübung. Diese Ergebnisse wurden per Computer gescannt (Abb. B).

### b) Eiweißbestimmung in menschlichen Blutserumlösungen

Der erfindungsgemäße Testkit wurde in Granulatform jeweils zu 6 Serum- und Urinproben und im Vergleich dazu zu 6 Urinproben mit reinem Albumin gegeben. Sie wiesen einen Eiweißgehalt von 0,02 bis 0,8 g/l auf:
Serum + Urinproben mit 0,02 g/l, 0,05 g/l, 0,1 g/l, 0,25 g/l, 0,5 g/l, 0,8 g/l Eiweißgehalt in der Mischung und
Urinproben mit 0,02 g/l, 0,05 g/l, 0,1 g/l, 0,25 g/l, 0,5 g/l, 0,8 g/l reinem Albumin.

Die Bestimmung von Eiweiß und Globulinen wurde wie unter a) beschrieben durchgeführt.

Ergebnis:
1. Nach 5 bis 10 Minuten waren in allen Proben Eiweißringe über dem Granulat zu sehen, d.h. Eiweiß war in allen Proben vorhanden.
2. In vier Serumproben waren deutliche Flocken 1 Stunde nach Schüttelung zu sehen, in zwei Proben (0,5 g/l und 0,8 g/l setzte sich nach 2 Stunden und in weiteren 2 Proben (0,1 g/l und 0,25 g/l) nach 4 Stunden ein typisches Sediment im Reagenzglas ab, d.h. alle Serumglobuline (α, β, γ) wurden in der Probe festgestellt. Dabei entsprach die Größe des Sediments der Höhe des Eiweißgehaltes.
3. Die Albuminproben zeigten alle lediglich eine homogene Trübe.

### c) Eiweißbestimmung in Urinproben von Patienten

Es wurden die Urinproben von 3 Diabetikern, von 2 Patienten mit Leukämien und von 11 Patienten mit Nierenerkrankungen untersucht. Allen diesen Proben wurde der Testkit in Granulatform zugegeben.

Ergebnisse:
1. In allen Urinproben wurden typische Eiweißablagerungen über den Granulaten festgestellt, d.h. Gesamteiweiß war vorhanden.
2. Nach dem Herstellen einer homogenen Lösung zeigte die Urinprobe von einem Leukämie-Patienten und von 3 Patienten mit Nierenerkrankungen zuerst nach 30-60 Minuten bedeutende Flocken und nach 3 Stunden ein typisches Sediment, d.h. in diesen Proben waren Globuline vorhanden.

Mit elektrophoretischen und immunologischen Methoden konnten diese Ergebnisse bestätigt werden.

### Beispiel 2

Der Testkit hatte die folgende Zusammensetzung:

| | |
|---|---|
| Sulfosalicylsäure | 75 Gew.% |
| NaCl | 4 Gew.% |
| Kupfersulfat | 1 Gew.% |
| Dextran | 12 Gew.% |
| D-Glucose | 8 Gew.% |

### a) Vergleichende Eiweißuntersuchungen von Urinproben mit Gesamteiweiß-(GE ) und erhöhtem alfa-1-Mikroglobulingehalt (a-mG ).

50 Urinproben mit bestimmtem Eiweißgehalt (von einem medizinischen Labor geliefert) wurden mit dem erfindungsgemäßen Testkit analysiert, in dem die Proben in ein Reagenzglas überführt wurden, in welchem sich der Testkit in fester fixierter Form mit der o.g. Zusammensetzung befand.

Ergebnis :
1. In allen 50 Proben waren nach 5 bis 10 Minuten Eiweißringe über der Reagenzmischung
   zu sehen,d.h.in jeder Probe ist GE vorhanden.
2. In 14 Fällen waren keine einzelnen Eiweißringe entstanden, sondern es hatten sich zwei Ringe über der Reagenzmischung gebildet, vgl. Abb.2. In den meisten Fällen hatten diese Urinproben einen erhöhten a-mG, d.h. ein erhöhtes Verhältniss von a-mG (mg) zu GE (1g). Die Tendenz - direkte Abhängigkeit des Verhältnisses von a-mG/GE und einem erhöhten Auftreten von Eiweißbildern mit zwei Ringen - wurde mittels Statistikanalyse bestätigt.

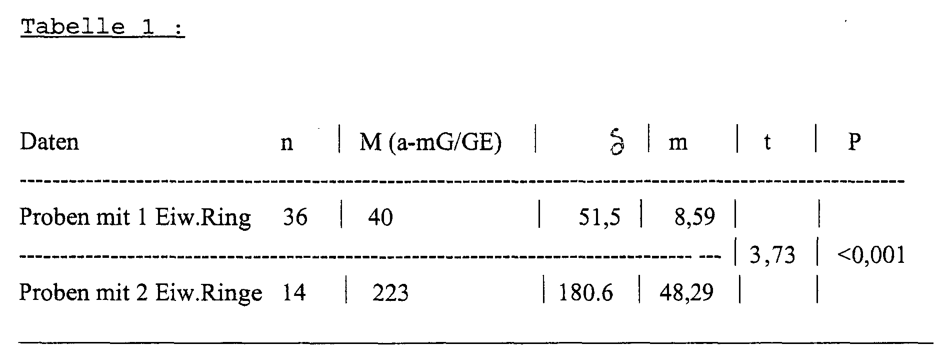

### b) Vergleichende Eiweißbestimmung reiner Proteinlösungen

Albumin aus bovinem Serum wurde im normalen Urin mit einer Konzentration von 2500 mg/l gelöst und jeweils 0,5 ml wurden in zwei Testreagenzgläser gegeben. In zwei andere Testreagenzgläser wurden jeweils 0,5 ml einer Mischungslösung (Albumin mit gleicher Konzentration und gamma-Globulin mit einer Konzentration von 100n mg/l gegeben und wie unter 2a) geprüft, wobei zusätzlich 0,1 ml Aceton zugegeben wurden.

Ergebnis :
1. In allen vier Proben waren nach 5 bis 10 Minuten Eiweißringe über dem Reagenz zu sehen, d.h. in jede Probe ist Eiweiß vorhanden.
2. In den Proben, die zusätzlich gamma-Globulin enthielten, verwandelte sich der Eiweißring nach 40 Minuten in einen sogenannten "Gebirgsring", d.h. das gamma-Globulinpräzipitat tauchte aus dem gesamten Eiweißniederschlag teilweise auf (Abb.3).

### c) Vergleichende Eiweißuntersuchungen von Urinproben mit erhöhtem Immunoglobulingehalt.

Es wurden 3 Urinproben mit einem festgesetzten, erhöhten Immunoglobulingehalt (mit den Konzentrationen von 115 mg/l, 260 mg/l, 290 mg/l), eine Urinprobe mit wenig Immunoglobulin (27 mg/l) und eine unbekannte Probe wie unter 2a) geprüft.

Ergebnis :
1. In allen fünf Proben waren nach 5 bis 10 Minuten Eiweißringe über dem Reagenz zu sehen, d.h. in jeder Probe ist Eiweiß vorhanden.
2. In den Proben mit einer hohen Immunoglobulinkonzentration und in der unbekannten Probe wurde nach 1,5 Stunden der sogenannte "Gebirgsring" beobachtet (wie unter 2b) beschrieben), vgl. Abb.4.

### d) Vergleichende Eiweißuntersuchungen von reiner Albuminlösung und Albumin-Hämoglobinmischungen sowie von Urin mit Mikrohämaturie.

Es wurden eine reine Albuminlösung (mit einer Konzentration von 1000 mg/l), eine Albuminhämoglobinmischung (ca.300-600 *µ*l Blut/l), sowie Urinproben mit und ohne Mikrohämaturie mit dem Testkit unter 2a) geprüft.

Ergebnis :
1. In allen Proben mit der Albumin-Hämoglobinmischung wurde nach 1 bis 1,5 Stunden ein roter Hämoglobinring über dem Reagenz (unter dem Albumin) beobachtet, vgl. Abb.5.
2. Ähnliche Ergebnisse wurden auch bei der Analyse von Urinproben mit Mikrohämaturie(ca von 300 Mkl Blut /l Urin) erhalten.

## Patentansprüche

1. Reagenzmischung als feste Formulierung zur Bestimmung von Gesamteiweiß und Globulinen in biologischen Flüssigkeitsproben,
**dadurch gekennzeichnet, dass**
sie neben üblichen Fällungsreagentien, die das Gesamteiweiß fällen und eine visuelle Beurteilung des Resultates der Fällungsreaktion sicherstellen, als weiteres Fällungsreagenz Kupfersulfat und zusätzlich mindestens ein Polysaccharid aufweist.

2. Reagenzmischung nach Anspruch 1,
**dadurch gekennzeichnet,**
**dass** in der festen Formulierung
| | |
|---|---|
| die Fällungsreagentien | mit 85-95 Gew.% und |
| das Polysaccharid | mit 5-15 Gew.% enthalten sind. |

3. Reagenzmischung nach Anspruch 1,
**dadurch gekennzeichnet,**
**dass** in der festen Formulierung zur zusätzlichen Bestimmung von Hämoglobin außerdem mindestens ein Monosaccharid enthalten ist.

4. Reagenzmischung nach Anspruch 3,
**dadurch gekennzeichnet,**
**dass** in der festen Formulierung
| | |
|---|---|
| die Fällungsreagentien | mit 70-90 Gew.%, |
| das Polysaccharid | mit 5-15 Gew.% und |
| das Monosaccharid | mit 5-15 Gew.% enthalten sind. |

5. Reagenzmischung nach Anspruch 1,
**dadurch gekennzeichnet, dass**
das Fällungsreagenz eine Mischung aus einer festen organischen Säure, einem anorganischen Salz und Kupfersulfat ist.

6. Reagenzmischung nach Anspruch 5,
**dadurch gekennzeichnet, dass**
die organische Säure zu 80-95 Gew.%, das anorganische Salz zu 4-15 Gew.% und Kupfersulfat mit 1-5 Gew.% enthalten sind.

7. Reagenzmischung nach Anspruch 1,
**dadurch gekennzeichnet, dass**
sie in Granulat- oder Tablettenform vorliegt.

8. Testkit zur Bestimmung von Gesamteiweiß und Globulinen in biologischen Flüssigkeitsproben, wobei der Testkit Reagentien enthält, die das Gesamteiweiß fällen und eine visuelle Beurteilung des Resultates der Fällungsreaktion sicherstellen,
**dadurch gekennzeichnet, dass**
er die Reagenzmischung in einer festen Formulierung umfasst, die neben den an sich üblichen Fällungsreagentien als weiteres Fällungsreagenz Kupfersulfat und zusätzlich mindestens ein Polysaccharid aufweist, wobei sich die feste Formulierung bereits im Testreagenzgefäß befindet, oder der Testkit das leere Testreagenzgefäß und die feste Formulierung lose in einem anderen Gefäß zur Verfügung stellt.

9. Verfahren zum Nachweis von Gesamteiweiß, Globulinen und gegebenenfalls von Hämoglobin in biologischen Flüssigkeitsproben,
**dadurch gekennzeichnet, dass** man
die zu untersuchende biologische Flüssigkeitsprobe mit der festen Reagenzmischung gemäß einem der Ansprüche 1-7 in Kontakt bringt und nach einer Einwirkungszeit von maximal 10 Minuten anhand vorhandener Eiweißringe auswertet, wobei man anhand des entstandenen Eiweißniederschlages über der festen Reagenzmischung den Gesamteiweißgehalt und bei Vorliegen eines zweiten Eiweißringes einen erhöhten Alpha-1-Mikroglobulingehalt bestimmt, anschließend den Testkit für weitere 30 Minuten bis 1,5 Stunden auf die biologische Flüssigkeit einwirken läßt und dann anhand des sich verändernden Präzipitatbildes Globuline und Hämoglobin bestimmt.

10. Verfahren nach Anspruch 9,
**dadurch gekennzeichnet, dass** man
zur Beschleunigung der Immunoglobulinpräzipitätsreaktion vor oder nach der Bestimmung des Gesamteiweißgehaltes Aceton zur Probe zugibt.
